Europäisches Patentamt

European Patent Office

Office européen des brevets

(19)

(11) **EP 1 169 636 B1**

(12) **FASCICULE DE BREVET EUROPEEN**

(45) Date de publication et mention
de la délivrance du brevet:
**21.06.2006   Bulletin 2006/25**

(21) Numéro de dépôt: **00910925.7**

(22) Date de dépôt: **13.03.2000**

(51) Int Cl.:
*G01N 29/04* (2006.01)        *G01N 29/06* (2006.01)
*A61B 8/08* (2006.01)

(86) Numéro de dépôt international:
**PCT/FR2000/000599**

(87) Numéro de publication internationale:
**WO 2000/055616 (21.09.2000 Gazette 2000/38)**

(54) **PROCEDE ET DISPOSITIF D'IMAGERIE UTILISANT LES ONDES DE CISAILLEMENT**

VERFAHREN UND VORRICHTUNG ZUR BILDERZEUGUNG DURCH VERWENDUNG VON
SCHERWELLEN

IMAGING METHOD AND DEVICE USING SHEAR WAVES

(84) Etats contractants désignés:
**AT BE CH CY DE DK ES FI FR GB GR IE IT LI LU
MC NL PT SE**

(30) Priorité:   **15.03.1999   FR 9903157**

(43) Date de publication de la demande:
**09.01.2002   Bulletin 2002/02**

(73) Titulaire: **Société d' Elastographie Impulsionnelle
pour les
Systèmes de Mesure de l'Elasticité ( SEISME)
75005 Paris (FR)**

(72) Inventeurs:
• **FINK, Mathias
92190 Meudon (FR)**

• **SANDRIN, Laurent
75013 Paris (FR)**
• **TANTER, Michael
75006 Paris (FR)**
• **CATHELINE, Stefan
35860 Bais (FR)**

(74) Mandataire: **Burbaud, Eric
Cabinet Plasseraud
65/67 rue de la Victoire
75440 Paris Cedex 09 (FR)**

(56) Documents cités:
**FR-A- 2 655 835        US-A- 5 099 848
US-A- 5 524 636        US-A- 5 810 731
US-A- 5 839 441**

EP 1 169 636 B1

**Description**

La présente invention est relative aux procédés et dispositifs d'imagerie utilisant les ondes de cisaillement.

Plus particulièrement, l'invention concerne un procédé d'imagerie utilisant les ondes de cisaillement pour observer un milieu viscoélastique diffusant qui contient des particules réfléchissant les ondes ultrasonores de compression, procédé dans lequel on génère une onde élastique de cisaillement dans le milieu viscoélastique et on observe, au moyen d'au moins une onde ultrasonore de compression, le déplacement du milieu viscoélastique soumis à ladite onde de cisaillement.

Le document US-A-5 810 731 décrit un exemple d'un tel procédé, dans lequel l'onde de cisaillement est générée localement à l'intérieur du milieu viscoélastique observé, au moyen de la pression de radiation d'une onde ultrasonore modulée et focalisée sur un point à observer. On envoie ensuite sur ce point focal une onde ultrasonore supplémentaire dont la réflexion permet de connaître certains paramètres de propagation de l'onde de cisaillement (notamment la viscosité dynamique du milieu et son module de cisaillement) au niveau du point focal susmentionné.

Cette technique présente l'inconvénient de permettre l'analyse d'un seul point du milieu viscoélastique étudié à chaque fois qu'une onde de cisaillement est générée. Si l'on veut obtenir une image complète du milieu viscoélastique observé, il est nécessaire de répéter l'opération un très grand nombre de fois, ce qui implique un temps de pause important (par exemple, plusieurs minutes) pour obtenir cette image.

Ce temps de pause important rend ce procédé de l'art antérieur peu pratique à utiliser.

De plus, un tel temps de pause peut nuire à l'utilisation dudit procédé pour obtenir une image d'un tissu vivant, qui est toujours en mouvement.

La présente invention a notamment pour but de pallier ces inconvénients.

A cet effet, selon l'invention, un procédé du genre en question est essentiellement caractérisé en ce qu'on génère l'onde de cisaillement en appliquant au milieu viscoélastique une excitation ayant la forme d'une impulsion basse fréquence qui présente une fréquence centrale f comprise entre 20 et 5000 Hz, cette impulsion basse fréquence présentant une durée comprise entre 1/2f et 20/f,

en ce qu'il comporte une étape d'observation de propagation au cours de laquelle on observe la propagation de l'onde de cisaillement simultanément en une multitude de points dans le milieu observé, ces points formant un champ d'observation sensiblement continu s'étendant au moins selon un premier axe, cette étape d'observation de propagation de l'onde de cisaillement consistant à :

- émettre dans le milieu observé une succession d'au moins 10 tirs d'ondes ultrasonores de compression à une cadence comprise entre 100 et 100 000 tirs par seconde,
- détecter et enregistrer en temps réel les échos générés par les particules réfléchissantes du milieu viscoélastique à chaque tir d'onde ultrasonore, ces échos correspondant (directement ou indirectement) à des images successives du milieu observé,

et en ce que ledit procédé comporte en outre une étape ultérieure de traitement d'image au cours de laquelle on traite en temps différé les images ainsi obtenues au moins par intercorrélation entre images successives, pour déterminer en chaque point du champ d'observation un paramètre de mouvement choisi entre le déplacement et la déformation du milieu viscoélastique, de façon à obtenir ainsi une succession d'images montrant l'évolution du paramètre de mouvement du milieu viscoélastique sous l'effet de la propagation de l'onde de cisaillement.

Grâce à ces dispositions, on obtient un film illustrant clairement la propagation de l'onde de cisaillement dans le milieu viscoélastique, qui peut permettre par exemple, dans des applications médicales, de repérer directement des zones cancéreuses dans les tissus d'un patient : la propagation des ondes de cisaillement s'y déroule en effet très différemment des zones voisines.

Ce repérage s'effectue beaucoup plus facilement que par une observation classique par simple échographie ultrasonore, puisque la propagation des ondes de cisaillement est fonction du module de cisaillement du milieu, lui-même très variable entre une zone de tissus sains et une zone de tissus cancéreux : le module de cisaillement varie typiquement dans un rapport de 1 à 30 entre une zone saine et une zone cancéreuse, alors que le module de compression, qui régit la propagation des ondes acoustiques de compression utilisées dans l'échographie ultrasonore, varie seulement de l'ordre de 5% entre un tissu sain et un tissu cancéreux.

On notera que le film obtenu illustre la propagation de l'onde de cisaillement beaucoup plus clairement que la simple succession des images données par les particules réfléchissantes du milieu, puisque ledit film permet de visualiser à chaque instant les zones du milieu observé qui subissent des mouvements de même ampleur du fait de la propagation de l'onde de cisaillement, alors que la succession des images des particules réfléchissantes ne permettrait de visualiser qu'un brouillard de points lumineux en mouvement.

Dans des modes de réalisation préférés du procédé selon l'invention, on peut éventuellement avoir recours en outre à l'une et/ou à l'autre des dispositions suivantes :

- la durée de l'impulsion basse fréquence est comprise entre 1/2f et 2/f ;
- la fréquence centrale de l'impulsion basse fréquence est comprise entre 30 et 1000 Hz ;
- le milieu viscoélastique observé est constitué par un corps vivant comprenant au moins un organe interne soumis à des mouvements impulsionnels, l'impulsion basse fréquence qui génère l'onde de cisaillement étant constituée par un mouvement impulsionnel dudit organe interne ;
- le milieu viscoélastique observé est délimité par une surface extérieure, et l'impulsion basse fréquence est appliquée au niveau de cette surface extérieure ;
- l'impulsion basse fréquence est appliquée par un moyen d'excitation choisi parmi :

  • une onde acoustique générée par au moins un transducteur acoustique,
  • et un choc généré localement par contact physique au niveau de la surface extérieure du milieu viscoélastique ;

- on émet les tirs d'ondes ultrasonores de compression et on détecte les échos générés par les particules réfléchissantes du milieu viscoélastique, au moyen d'une batterie de transducteurs qui comprend au moins un transducteur et qui est disposée au contact de la surface extérieure du milieu viscoélastique, l'onde de cisaillement étant appliquée au milieu viscoélastique en imposant un déplacement impulsionnel à ladite batterie de transducteurs ;
- ledit paramètre de mouvement est la déformation du milieu viscoélastique : cette disposition est particulièrement utile dans le dernier cas envisagé ci-dessus, puisqu'elle permet de s'affranchir du déplacement de la batterie de transducteurs, déplacement qui perturberait sinon la mesure du déplacement des points du champ d'observation ;
- au cours de l'observation de la propagation de l'onde de cisaillement, on émet entre 100 et 10 000 tirs d'ondes ultrasonores de compression à une cadence comprise entre 100 et 100 000 tirs par seconde ;
- le champ d'observation s'étend au moins selon un plan comprenant d'une part, le premier axe et d'autre part, un deuxième axe perpendiculaire au premier axe ;
- au cours de l'étape d'observation de propagation, on utilise une batterie de plusieurs transducteurs acoustiques disposés au moins selon le deuxième axe pour émettre les tirs d'ondes ultrasonores de compression et détecter les échos générés par les particules réfléchissantes du milieu viscoélastique, les échos détectés par chaque transducteur acoustique étant mémorisés directement sans traitement préalable au cours de l'étape d'observation de propagation, et l'étape de traitement d'image comprenant une sous-étape préliminaire de formation de voies au cours de laquelle on génère une image du milieu viscoélastique correspondant à chaque tir d'onde ultrasonore de compression, par combinaison d'au moins certains des échos reçus par les différents transducteurs ;
- l'étape de traitement d'images est suivie (immédiatement ou non) par une étape de visualisation au cours de laquelle on visualise au ralenti un film constitué par la succession des images traitées, chaque point de chaque image présentant un paramètre optique qui varie suivant la valeur du paramètre de mouvement affecté à ce point ;
- le paramètre optique est choisi parmi le niveau de gris et le niveau chromatique ;
- l'étape de traitement d'images est suivie (immédiatement ou non) par une étape de cartographie au cours de laquelle, à partir de l'évolution du paramètre de mouvement au cours du temps dans le champ d'observation, on calcule au moins un paramètre de propagation de l'onde de cisaillement en au moins certains points du champ d'observation ;
- le paramètre de propagation de l'onde de cisaillement qui est calculé au cours de l'étape de cartographie est choisi parmi la vitesse des ondes de cisaillement, le module de cisaillement, l'atténuation des ondes de cisaillement, l'élasticité de cisaillement, et la viscosité de cisaillement.

Par ailleurs, l'invention a également pour objet un dispositif d'imagerie utilisant les ondes de cisaillement pour observer un milieu viscoélastique diffusant qui contient des particules réfléchissant les ondes ultrasonores de compression, ce dispositif comprenant des moyens d'excitation pour générer une onde élastique de cisaillement dans le milieu viscoélastique et des moyens d'acquisition pour observer, au moyen d'au moins une onde ultrasonore de compression, le déplacement du milieu viscoélastique soumis à ladite onde de cisaillement,
caractérisé en ce que les moyens d'excitation sont adaptés pour appliquer au milieu viscoélastique une excitation ayant la forme d'une impulsion basse fréquence qui présente une fréquence centrale f comprise entre 20 et 5000 Hz, cette impulsion basse fréquence présentant une durée comprise entre 1/2f et 20/f,
en ce que les moyens d'acquisition sont adaptés pour observer la propagation de l'onde de cisaillement simultanément en une multitude de points dans le milieu observé, ces points formant un champ d'observation sensiblement continu s'étendant au moins selon un premier axe, lesdits moyens d'acquisition étant adaptés pour :

- émettre dans le milieu observé une succession d'au moins 10 tirs d'ondes ultrasonores de compression à une cadence comprise entre 100 et 100 000 tirs par seconde,
- détecter et enregistrer en temps réel les échos générés par les particules réfléchissantes du milieu viscoélastique à chaque tir d'onde ultrasonore, ces échos correspondant (directement ou indirectement) à des images successives du milieu observé,

et en ce que ledit dispositif comporte en outre des moyens de traitement d'image adaptés pour traiter en temps différé les images obtenues à partir des moyens d'observation, au moins par intercorrélation entre images successives, pour déterminer en chaque point du champ d'observation un paramètre de mouvement choisi entre le déplacement et la déformation du milieu viscoélastique, de façon à obtenir ainsi une succession d'images montrant l'évolution du paramètre de mouvement du milieu viscoélastique sous l'effet de la propagation de l'onde de cisaillement.

Dans des modes de réalisation préférés du dispositif selon l'invention, on peut éventuellement avoir recours en outre à l'une et/ou à l'autre des dispositions suivantes :

- les moyens d'observation comprennent une batterie de transducteurs qui inclut au moins un transducteur et qui est adaptée pour être disposée au contact d'une surface extérieure délimitant le milieu viscoélastique, les moyens d'excitation étant adaptés pour imposer un déplacement impulsionnel à ladite batterie de transducteurs ;
- ledit paramètre de mouvement est la déformation du milieu viscoélastique ;
- le champ d'observation s'étend au moins selon un plan comprenant d'une part, le premier axe et d'autre part, un deuxième axe perpendiculaire au premier axe, la batterie de transducteurs comportant plusieurs transducteurs disposés au moins selon le deuxième axe, des moyens de commande étant prévus pour sélectivement faire fonctionner le dispositif soit dans le mode d'imagerie par ondes de cisaillement, soit dans un mode d'échographie standard permettant d'acquérir entre 10 et 100 images par seconde.

D'autres caractéristiques et avantages de l'invention apparaîtront au cours de la description suivante de plusieurs de ses formes de réalisation, données à titre d'exemples non limitatifs, en regard des dessins joints.

Sur les dessins :

- la figure 1 est une vue schématique d'un dispositif d'imagerie par ondes de cisaillement selon une forme de réalisation de l'invention,
- et la figure 2 est une vue de détail montrant une variante du dispositif de la figure 1.

Sur les différentes figures, les mêmes références désignent des éléments identiques ou similaires.

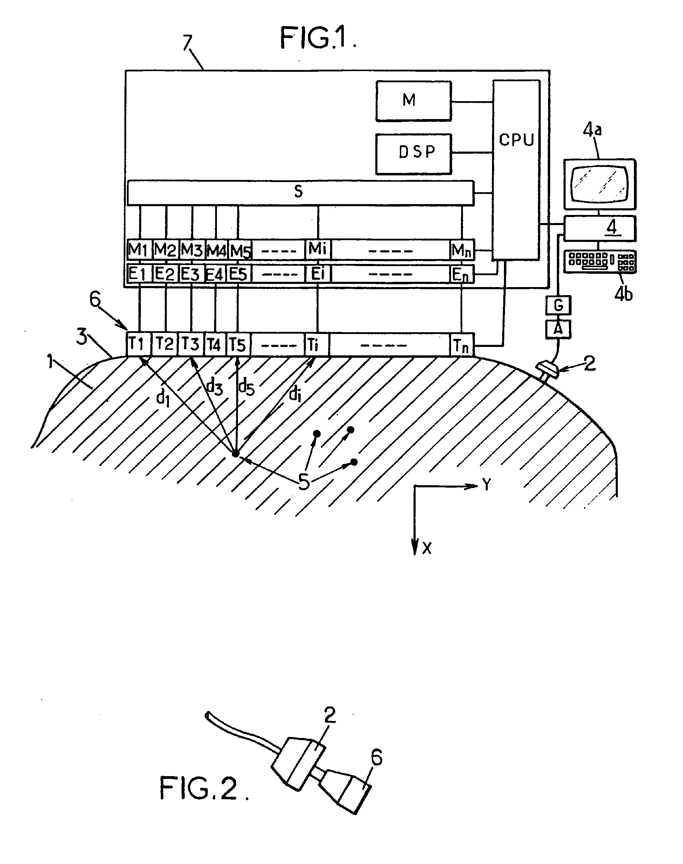

La figure 1 représente un exemple de dispositif d'imagerie par ondes de cisaillement selon l'invention, pour étudier la propagation des ondes élastiques de cisaillement dans un milieu viscoélastique 1 qui est diffusant vis à vis des ondes ultrasonores de compression, et qui peut être par exemple :

- un corps inerte, notamment dans le cas du contrôle de qualité pour des applications industrielles, notamment agro-alimentaires,
- ou un corps vivant, par exemple une partie du corps d'un patient, dans le cas des applications médicales.

Ce dispositif comporte un transducteur acoustique tel qu'un haut-parleur 2 ou un pot vibreur qui est disposé contre la surface extérieure 3 du milieu observé 1, cette surface 3 étant constituée par exemple par la peau du patient dans les applications médicales.

Le haut-parleur 2 peut être commandé par un _micro-ordinateur 4, par exemple par l'intermédiaire d'un circuit générateur d'impulsions basse fréquence G (ce circuit peut être constitué notamment par la carte son du micro-ordinateur 4) et d'un amplificateur A, pour appliquer à la surface 3 du milieu observé une excitation sous la forme d'une impulsion basse fréquence, de façon à générer une onde de cisaillement dans le milieu viscoélastique 1.

Cette impulsion basse fréquence présente en général une amplitude pouvant être de l'ordre de 1 mm et une fréquence centrale f comprise entre 20 et 5000 Hz, appliquée pendant une durée comprise entre $1/2f$ et $20/f$. De préférence, la durée d'application de l'impulsion basse fréquence est comprise entre $1/2f$ et $2/f$ et la fréquence f est comprise entre 30 et 1000 Hz, cette fréquence pouvant typiquement être de l'ordre de 50 Hz.

En variante l'onde acoustique de cisaillement pourrait également être obtenue (avec les caractéristiques susmentionnées d'amplitude et de fréquence) :

- par un choc généré localement par contact physique au niveau de la surface extérieure du milieu viscoélastique, par au moins un actionneur mécanique automatique commandé par le micro-ordinateur 4,
- par un choc appliqué manuellement par contact physique au niveau de la surface extérieure du milieu viscoélastique,
- ou encore, par un mouvement impulsionnel naturel d'un organe interne du corps humain ou animal (par exemple un battement du coeur) dans les applications médicales.

L'onde élastique de cisaillement produite par le haut-parleur 2 se déplace avec une vitesse Cs relativement faible, de l'ordre de quelques m/s (typiquement, de 1 à 10 m/s dans le corps humain) en produisant des mouvements internes dans le milieu viscoélastique 1 observé.

Ces mouvements sont suivis en envoyant dans le milieu 1, des ondes ultrasonores de compression qui interagissent avec les particules diffusantes 5 contenues dans le milieu 1, lesquelles particules sont réfléchissantes pour les ondes ultrasonores de compression. Les particules 5 peuvent être constituées par toute hétérogénéité du milieu 1, et notamment, lorsqu'il s'agit d'une application médicale, par des particules de collagène présentes dans les tissus humains.

Pour observer la propagation de l'onde de cisaillement, on utilise donc une sonde ultrasonore 6 disposée contre la surface extérieure 3 du milieu observé 1. Cette sonde envoie, selon un axe X, des impulsions d'ondes ultrasonores de compression du type de celles couramment utilisées en échographie, à une fréquence comprise par exemple entre 1 et 100 MHz et de préférence entre 3 et 15 MHz. On notera que la sonde 6 peut être disposée :

- soit du même côté du milieu 1 que le haut-parleur 2, comme représenté sur la figure 1,
- soit à l'opposé du haut-parleur 2 par rapport au milieu 1,
- soit dans toute autre position, par exemple dans une disposition transversale par rapport au haut-parleur 2.

La sonde ultrasonore 6 est constituée par une batterie de n transducteurs ultrasonores T1, T2, ..., Ti, ..., Tn, n étant un nombre entier n au moins égal à 1.

Cette sonde 6 se présente le plus souvent sous la forme d'une barrette linéaire pouvant comprendre par exemple n = 128 transducteurs alignés selon un axe Y perpendiculaire à l'axe X, envoyant simultanément leurs impulsions d'ondes ultrasonores de façon à générer une onde "plane" (c'est à dire en l'occurrence une onde dont le front d'onde est rectiligne dans le plan X, Y) ou tout autre type d'onde éclairant l'ensemble du champ d'observation.

En variante, la batterie 2 de transducteurs peut éventuellement être réduite à un transducteur unique T1, ou au contraire se présenter sous la forme d'un réseau à deux dimensions s'étendant par exemple selon un plan perpendiculaire à l'axe X.

Chacun des transducteurs T1, T2, ... Tn est commandé par le micro-ordinateur 4 ou par une unité centrale CPU (qui est contenue par exemple dans une baie électronique 7 reliée par un câble souple à la sonde 6), pour émettre des tirs successifs d'ondes de compression ultrasonores dans le milieu 2, au cours d'une phase d'observation qui peut durer par exemple moins d'une seconde et au cours de laquelle sont émis p tirs d'ondes ultrasonores de compression (p étant un entier compris entre 100 et 10 000 et de préférence entre 1000 et 100 000), à une cadence comprise entre 100 et 100 000 tirs par seconde et de préférence comprise entre 1000 et 100 000 tirs par seconde, notamment entre 1000 et 10 000 tirs par seconde (cette cadence est limitée par le temps d'aller-retour de l'onde de compression dans le milieu 1, donc par l'épaisseur du milieu 1 dans la direction X : il faut en effet que tous les échos générés par l'onde de compression aient été reçus par la sonde 6 avant d'envoyer une nouvelle onde de compression).

Les tirs d'ondes ultrasonores de compression de la phase d'observation commencent de préférence juste avant l'émission de l'onde de cisaillement.

De plus, dans le cas où l'onde de cisaillement est générée par un mouvement impulsionnel d'un organe d'un corps vivant, on peut avantageusement synchroniser le démarrage des tirs d'ondes ultrasonores de compression avec ce mouvement impulsionnel par exemple dans le cas où l'onde de cisaillement est générée par un battement de coeur, on peut synchroniser le démarrage des tirs d'ondes ultrasonores de compression avec une phase choisie de l'électrocardiogramme.

Chacun de ces tirs donne lieu à la propagation d'une onde ultrasonore de compression dans le milieu 1, avec une vitesse de propagation beaucoup plus élevée que les ondes de cisaillement, par exemple de l'ordre de 1500 m/s dans le corps humain.

L'onde ultrasonore ainsi générée intéragit avec les particules réfléchissantes 5, ce qui génère des échos ou autres perturbations analogues du signal, connus en soi sous le nom de "bruits de speckle" dans le domaine de l'échographie.

Ces "bruits de speckle" sont captés par les transducteurs T1, ... ,Tn après chaque tir. Le signal sij(t) ainsi capté par chaque transducteur Ti après le tir n° j est tout d'abord échantillonné à haute fréquence (par exemple de 30 à 100 MHz) et numérisé en temps réel (par exemple sur 8 bits, ou dans certains cas sur 1 bit) par un échantillonneur appartenant à la baie 7 et relié à ce transducteur, respectivement E1, E2, ... En.

Le signal sij(t) ainsi échantillonné et numérisé est ensuite mémorisé, également en temps réel, dans une mémoire Mi appartenant à la baie 7 et propre au transducteur Ti.

Chaque mémoire Mi présente par exemple une capacité de l'ordre de 1 Mo, et contient l'ensemble des signaux sij(t) reçus successivement pour les tirs j = 1 à p.

En temps différé, après la mémorisation de tous les signaux sij (t) correspondant à une même propagation d'onde de cisaillement, l'unité centrale CPU fait retraiter ces signaux par un circuit sommateur S appartenant à la baie 7 (ou bien elle effectue elle-même ce traitement, ou encore ledit traitement peut être effectué dans le micro-ordinateur 4), par un processus classique de formation de voies.

On génère ainsi des signaux Sj (x, y) qui correspondent chacun à l'image du champ d'observation après le tir n˚ j.

Par exemple, on peut déterminer un signal Sj (t) par la formule suivante :

$$Sj(t) = \sum_{i=1}^{n} \alpha_i(x,y).sij[t(x,y) + d_i(x,y)/V]$$

où :

- sij est le signal brut perçu par le transducteur n˚ i après le tir d'onde ultrasonore de compression n˚ j,
- t (x,y) est le temps mis par l'onde ultrasonore de compression pour atteindre le point du champ d'observation de coordonnées (x,y), avec t = 0 au début du tir n˚ j,
- di(x,y) est la distance entre le point du champ d'observation de coordonnées (x,y) et le transducteur n˚ i, ou une approximation de cette distance,
- V est la vitesse moyenne de propagation des ondes acoustiques ultrasonores de compression dans le milieu viscoélastique observé.
- et $\alpha i(x,y)$ est un coefficient de pondération tenant compte de lois d'apodisation (en pratique, on pourra dans de nombreux cas considérer que $\alpha i(x,y)=1$).

La formule ci-dessus s'applique mutatis mutandis lorsque le champ d'observation est à 3 dimensions (réseau plan de transducteurs), en remplaçant les coordonnées spatiales (x,y) par (x,y,z).

Lorsque la sonde 6 comporte un seul transducteur, l'étape de formation de voies est inutile, et on a directement Sj(x) = sj[2.x/V], avec les mêmes notations que ci-dessus.

Après l'étape éventuelle de formation de voies, l'unité centrale CPU mémorise dans une mémoire centrale M appartenant à la baie 7 les signaux d'images Sj(x,y) ou Sj(x) ou Sj(x,y,z), qui correspondent chacun au tir n˚ j. Ces signaux peuvent également être mémorisés dans le micro-ordinateur 4 lorsqu'il effectue lui-même le traitement d'image.

Ces images sont ensuite traitées deux à deux, ou-jours en temps différé, par intercorrélation. Cette intercorrélation peut être réalisée dans un circuit DSP appartenant à la baie 7, ou être programmée dans l'unité centrale CPU ou dans le micro-ordinateur 4.

A titre d'exemple, cette intercorrélation peut se faire en comparant les signaux Sj (x,y) et Sj+1 (x,y) (dans le cas d'un champ d'observation à 2 dimensions) sur des fenêtres spatiales glissantes de longueur $\Delta$x prédéterminée, pouvant aller par exemple de $\lambda$ à 10$\lambda$, où $\lambda$ est la longueur d'onde des ondes ultrasonores de compression (soit environ 0,42 à 4,2 mm à 3,5 MHz dans l'eau ou le corps humain). De plus, les fenêtres susmentionnées peuvent se recouvrir l'une l'autre sur environ 20% de leur longueur selon l'axe X.

Au cours de ce processus d'intercorrélation, on maximise une fonction d'intercorrélation <Sj(x,y),Sj+1(x,y)> afin de déterminer le déplacement subi par chaque particule 5 donnant lieu à un écho ultrasonore, dans la direction X.

Des exemples de tels calculs d'intercorrélation sont donnés dans l'état de la technique, notamment par O'Donnell et al. (« Internal displacement and strain imaging using speckle tracking », IEEE transactions on ultrasonic, ferroelectrics, and frequency control, vol. 41, n˚ 3, mai 1994, p. 314-325) et par Ophir et al. (« Elastography a quantitative method for imaging the elasticity of biological tissues », Ultrasonic imag., vol. 13, p.111-134, 1991).

On obtient ainsi une succession de champs de déplacements Djx(x,y) du milieu 1 sous l'effet de l'onde de cisaillement, dans la direction X.

Cette succession de champs de déplacements est stockée dans la mémoire M ou dans le micro-ordinateur 4 et peut être visualisée, notamment au moyen de l'écran 4a du micro-ordinateur, sous la forme d'un film ralenti où la valeur des déplacements est illustrée par un paramètre optique tel que par un niveau de gris ou par un niveau chromatique.

On visualise ainsi parfaitement les différences de propagation de l'onde de cisaillement entre les zones de caractéristiques différentes du milieu 1, par exemple les tissus sains et les tissus cancéreux dans le cas d'une application médicale.

Ce film de propagation de l'onde de cisaillement est en outre superposable avec une image échographique classique, qui peut être générée par le dispositif décrit ci-dessus, lequel est apte à fonctionner :

- soit dans le mode d'imagerie par ondes de cisaillement,
- soit dans un mode d'échographie standard, en fonction des commandes reçues par exemple depuis le clavier 4b du micro-ordinateur.

Par ailleurs, il est également possible de calculer non pas les déplacements de chaque point du milieu observé 1, mais les déformations Ejx (x,y) du milieu dans la direction X, c'est à dire les dérivées des déplacements Djx(x,y) par rapport à x.

Ces champs de déformations successifs sont utilisables comme précédemment pour visualiser clairement la propagation de l'onde de cisaillement sous la forme d'un film, et présentent en outre l'avantage de s'affranchir des déplacements de la sonde 6 par rapport au milieu observé 1.

Cette variante est particulièrement intéressante dans la forme de réalisation de la figure 2, où la sonde 6 est portée par le haut-parleur ou vibreur 2, ce qui implique forcément des mouvements de ladite sonde puisque c'est elle-même qui génère alors l'onde de cisaillement.

A partir des champs de déplacements ou de déformations, on peut le cas échéant procéder en outre à une étape de cartographie au cours de laquelle, à partir de l'évolution du paramètre de mouvement (déplacement ou déformation) au cours du temps dans le champ d'observation X, Y (ou X dans la ces d'un seul transducteur, ou X, Y, Z dans le cas d'un réseau plan de transducteurs), on calcule au moins un paramètre de propagation de l'onde de cisaillement, soit en certains points du champ d'observation choisis par l'utilisateur à partir du micro-ordinateur 4, soit dans tout le champ d'observation.

Le paramètre de propagation de l'onde de cisaillement qui est calculé au cours de l'étape de cartographie est choisi par exemple parmi la vitesse Cs des ondes de cisaillement, le module de cisaillement $\mu$, l'atténuation $\alpha$ des ondes de cisaillement, l'élasticité de cisaillement $\mu$1viscosité de cisaillement $\mu$2.

Ce calcul est effectué par un processus classique d'inversion, dont un exemple est donné ci-après dans le cas d'un champ d'observation à deux dimensions (le même processus s'appliquerait mutatis mutandis dans le cas d'un champ d'observation à une ou à trois dimensions, respectivement pour un seul transducteur T1 ou pour un réseau plan de transducteurs).

Dans cet exemple on fera l'approximation que la viscosité de cisaillement $\mu$2 est nulle et que le milieu est isotrope.

L'équation d'onde donnant le vecteur déplacement D de chaque point du milieu 1 s'écrit :

$$\rho \frac{\partial^2 \vec{D}}{\partial t^2} = \Delta(\mu.\vec{D}) \quad (\mathrm{I}),$$

où $\rho$ est la densité du milieu 1, $\mu$ est le module de cisaillement (par hypothèse réduit à sa partie réelle l'élasticité de cisaillement $\mu$1 puisque la viscosité de cisaillement $\mu$2 est supposée nulle).

Pour la première composante u du vecteur D, c'est à dire pour le déplacement du milieu 1 dans la direction X, on a donc :

$$\rho \frac{\partial^2 u}{\partial t^2} = \frac{\partial^2 (\mu u)}{\partial x^2} + \frac{\partial^2 (\mu u)}{\partial y^2} \quad (\mathrm{II}).$$

Après transformée de Fourier temporelle et discrétisation, cette équation peut être écrite sous la forme matricielle suivante, qui peut être écrite pour chaque fréquence du spectre de l'onde de cisaillement :

$$\vec{B} = H.\vec{M} \quad (\mathrm{III}),$$

- M est un vecteur de dimension (L+2).(M+2)-4 dont chaque composante vaut $\mu_{1m}$, c'est à dire la valeur locale du module de cisaillement en chaque point discrétisé de coordonnées $(x_1, y_m)$, où 1 et m sont des entiers compris entre 0 et respectivement L+1 et M+1 en éliminant les couples (I,m) valant (0,0), (0,M+1), (L+1,0) et (L+1,M+1), L+2 et M+2 étant les nombres de points discrétisés dans l'image du milieu 1 respectivement selon les axes X et Y,
- B est un vecteur de dimension L.M, dont les composantes valent $-\overline{\omega}^2.\rho.U_{1m}$ où $\overline{\omega}$ est la pulsation de l'onde de cisaillement basse fréquence, $\rho$ est la densité du milieu, $U_{1m}$ est la transformée de Fourier temporelle du déplacement u au point de coordonnées $(x_1, y_m)$, l étant compris entre 1 et L et m étant compris entre 1 et M,
- et H est une matrice de dimension L.M lignes sur (L+2).(M+2)-4 colonnes, dont les composantes sont également toutes connues à partir de l'équation d'onde.

En juxtaposant un nombre suffisant d'équations (III) correspondant respectivement à différentes disponibles du spectre de fréquences de l'onde de cisaillement, on obtient une équation matricielle globale qui peut être résolue par inversion matricielle, pour obtenir le vecteur M, c'est à dire la valeur du module de cisaillement $\mu$ en tout point du champ d'observation.

On peut alors en déduire éventuellement la valeur locale de la vitesse de propagation Cs de l'onde de cisaillement en chaque point, par la formule :

$$Cs = \sqrt{\frac{\mu}{\rho}} \ .$$

Le mode de calcul serait le même en utilisant non plus les déplacements, mais les déformations du milieu observé 1.

**Revendications**

**1.**   Procédé d'imagerie utilisant les ondes de cisaillement pour observer un milieu viscoélastique diffusant (1) qui contient des particules (5) réfléchissant les ondes ultrasonores de compression, procédé dans lequel on génère une onde élastique de cisaillement dans le milieu viscoélastique et on observe, au moyen d'au moins une onde ultrasonore de compression, le déplacement du milieu viscoélastique (1) soumis à ladite onde de cisaillement,
**caractérisé en ce qu'**on génère l'onde de cisaillement en appliquant au milieu viscoélastique une excitation ayant la forme d'une impulsion basse fréquence qui présente une fréquence centrale f comprise entre 20 et 5000 Hz, cette impulsion basse fréquence présentant une durée comprise entre 1/2f et 20/f,
**en ce qu'**il comporte une étape d'observation de propagation au cours de laquelle on observe la propagation de l'onde de cisaillement simultanément en une multitude de points dans le milieu observé, ces points formant un champ d'observation sensiblement continu s'étendant au moins selon un premier axe (X), cette étape d'observation de propagation de l'onde de cisaillement consistant à :

  - émettre dans le milieu observé une succession d'au moins 10 tirs d'ondes ultrasonores de compression à une cadence comprise entre 100 et 100 000 tirs par seconde,
  - détecter et enregistrer en temps réel les échos générés par les particules réfléchissantes du milieu viscoélastique à chaque tir d'onde ultrasonore, ces échos correspondant à des images successives du milieu observé,

et **en ce que** ledit procédé comporte en outre une étape ultérieure de traitement d'image au cours de laquelle on traite en temps différé les images ainsi obtenues au moins par intercorrélation entre images successives, pour déterminer en chaque point du champ d'observation un paramètre de mouvement choisi entre le déplacement et la déformation du milieu viscoélastique, de façon à obtenir ainsi une succession d'images montrant l'évolution du paramètre de mouvement du milieu viscoélastique sous l'effet de la propagation de l'onde de cisaillement.

**2.**   Procédé selon la revendication 1, dans lequel la durée de l'impulsion basse fréquence est comprise entre 1/2f et 2/f.

**3.**   Procédé selon la revendication 1 ou la revendication 2, dans lequel la fréquence centrale de l'impulsion basse fréquence est comprise entre 30 et 1000 Hz.

**4.**   Procédé selon l'une quelconque des revendications précédentes, dans lequel le milieu viscoélastique (1) observé est constitué par un corps vivant comprenant au moins un organe interne soumis à des mouvements impulsionnels, l'impulsion basse fréquence qui génère l'onde de cisaillement étant constituée par un mouvement impulsionnel dudit organe interne.

**5.**   Procédé selon l'une quelconque des revendications 1 à 3, dans lequel le milieu viscoélastique (1) observé est délimité par une surface extérieure (3), et l'impulsion basse fréquence est appliquée au niveau de cette surface extérieure.

**6.**   Procédé selon la revendication 5, dans lequel l'impulsion basse fréquence est appliquée par un moyen d'excitation choisi parmi :

  - une onde acoustique générée par au moins un transducteur acoustique (2),

- et un choc généré localement par contact physique au niveau de la surface extérieure (3) du milieu viscoélastique.

**7.** Procédé selon la revendication 5 ou la revendication 6, dans lequel on émet les tirs d'ondes ultrasonores de compression et on détecte les échos générés par les particules réfléchissantes du milieu viscoélastique, au moyen d'une batterie de transducteurs (6) qui comprend au moins un transducteur (T1, ..., Tn) et qui est disposée au contact de la surface extérieure du milieu viscoélastique, l'onde de cisaillement étant appliquée au milieu viscoélastique en imposant un déplacement impulsionnel à ladite batterie de transducteurs.

**8.** Procédé selon la revendication 7, dans lequel ledit paramètre de mouvement est la déformation du milieu viscoélastique.

**9.** Procédé selon l'une quelconque des revendications précédentes, dans lequel, au cours de l'observation de la propagation de l'onde de cisaillement, on émet entre 100 et 10 000 tirs d'ondes ultrasonores de compression à une cadence comprise entre 1000 et 100 000 tirs par seconde.

**10.** Procédé selon l'une quelconque des revendications précédentes, dans lequel le champ d'observation s'étend au moins selon un plan comprenant d'une part, le premier axe (X) et d'autre part, un deuxième axe perpendiculaire au premier axe (Y).

**11.** Procédé selon la revendication 10, dans lequel, au cours de l'étape d'observation de propagation, on utilise une batterie (6) de plusieurs transducteurs acoustiques (T1, ..., Tn) disposés au moins selon le deuxième axe (Y) pour émettre les tirs d'ondes acoustiques ultrasonores de compression et détecter les échos générés par les particules réfléchissantes (5) du milieu viscoélastique, les échos détectés par chaque transducteur acoustique étant mémorisés directement sans traitement préalable au cours de l'étape d'observation de propagation, et l'étape de traitement d'image comprenant une sous-étape préliminaire de formation de voies au cours de laquelle on génère une image du milieu viscoélastique correspondant à chaque tir d'onde ultrasonore de compression, par combinaison d'au moins certains des échos reçus par les différents transducteurs (T1, ..., Tn).

**12.** Procédé selon l'une quelconque des revendications précédentes, dans lequel l'étape de traitement d'images est suivie par une étape de visualisation au cours de laquelle on visualise au ralenti un film constitué par la succession des images traitées, chaque point de chaque image présentant un paramètre optique qui varie suivant la valeur du paramètre de mouvement affecté à ce point.

**13.** Procédé selon la revendication 12, dans lequel le paramètre optique est choisi parmi le niveau de gris et le niveau chromatique.

**14.** Procédé selon l'une quelconque des revendications précédentes, dans lequel l'étape de traitement d'images est suivie par une étape de cartographie au cours de laquelle, à partir de l'évolution du paramètre de mouvement au cours du temps dans le champ d'observation, on calcule au moins un paramètre de propagation de l'onde de cisaillement en au moins certains points du champ d'observation.

**15.** Procédé selon la revendication 14, dans lequel le paramètre de propagation de l'onde de cisaillement qui est calculé au cours de l'étape de cartographie est choisi parmi la vitesse des ondes de cisaillement, le module de cisaillement, l'atténuation des ondes de cisaillement, l'élasticité de cisaillement, et la viscosité de cisaillement.

**16.** Dispositif d'imagerie utilisant les ondes de cisaillement pour observer un milieu viscoélastique diffusant (1) qui contient des particules (5) réfléchissant les ondes ultrasonores de compression, ce dispositif comprenant des moyens d'excitation (2) pour générer une onde élastique de cisaillement dans le milieu viscoélastique et des moyens d'acquisition (CPU, Ti, Ei, Mi) pour observer, au moyen d'au moins une onde ultrasonore de compression, le déplacement du milieu viscoélastique soumis à ladite onde de cisaillement,
**caractérisé en ce que** les moyens d'excitation (2) sont adaptés pour appliquer au milieu viscoélastique (1) une excitation ayant la forme d'une impulsion basse fréquence qui présente une fréquence centrale f comprise entre 20 et 5000 Hz, cette impulsion basse fréquence présentant une durée comprise entre 1/2f et 20/f,
**en ce que** les moyens d'acquisition (CPU, Ti, Ei, Mi) sont adaptés pour observer la propagation de l'onde de cisaillement simultanément en une multitude de points dans le milieu observé, ces points formant un champ d'observation sensiblement continu s'étendant au moins selon un premier axe (X), lesdits moyens d'acquisition étant adaptés pour :

- émettre dans le milieu observé une succession d'au moins 10 tirs d'ondes ultrasonores de compression à une cadence comprise entre 100 et 100 000 tirs par seconde,
- détecter et enregistrer en temps réel les échos générés par les particules réfléchissantes du milieu viscoélastique à chaque tir d'onde ultrasonore, ces échos correspondant à des images successives du milieu observé,

et **en ce que** ledit dispositif comporte en outre des moyens de traitement d'image (CPU, S, DSP) adaptés pour traiter en temps différé les images obtenues à partir des moyens d'observation , au moins par intercorrélation entre images successives, pour déterminer en chaque point du champ d'observation un paramètre de mouvement choisi entre le déplacement et la déformation du milieu viscoélastique, de façon à obtenir ainsi une succession d'images montrant l'évolution du paramètre de mouvement du milieu viscoélastique sous l'effet de la propagation de l'onde de cisaillement.

**17.** Dispositif selon la revendication 16, dans lequel les moyens d'observation comprennent une batterie (6) de transducteurs (T1, ..., Tn) qui inclut au moins un transducteur et qui est adaptée pour être disposée au contact d'une surface extérieure (3) délimitant le milieu viscoélastique (1), les moyens d'excitation (2) étant adaptés pour imposer un déplacement impulsionnel à ladite batterie (6) de transducteurs.

**18.** Dispositif selon la revendication 17, dans lequel ledit paramètre de mouvement est la déformation du milieu viscoélastique.

**19.** Dispositif selon l'une quelconque des revendications 17 et 18, dans lequel le champ d'observation s'étend au moins selon un plan comprenant d'une part, le premier axe (X) et d'autre part, un deuxième axe (Y) perpendiculaire au premier axe, la batterie (6) de transducteurs comportant plusieurs transducteurs (T1, ..., Tn) disposés au moins selon le deuxième axe, des moyens de commande (4, CPU) étant prévus pour sélectivement faire fonctionner le dispositif soit dans le mode d'imagerie par ondes de cisaillement, soit dans un mode d'échographie standard permettant d'acquérir entre 10 et 100 images par seconde.

**Claims**

**1.** A method of imaging using shear waves to observe a diffusing viscoelastic medium (1) which contains particles (5) reflecting ultrasonic compression waves, in which method an elastic shear wave is generated in the viscoelastic medium and the displacement of the viscoelastic medium (1) subjected to said shear wave is observed by means of at least one ultrasonic compression wave,
**characterized in that** the shear wave is generated by applying to the viscoelastic medium an excitation having the form of a low-frequency pulse which exhibits a central frequency f of between 20 and 5000 Hz, this low-frequency pulse exhibiting a duration of between 1/2f and 20/f,
**in that** it comprises a propagation observation step in the course of which the propagation of the shear wave is observed simultaneously at a multitude of points in the observed medium, these points forming a substantially continuous observation field extending at least along a first axis (X), this shear wave propagation observation step consisting in:

- emitting into the observed medium a succession of at least 10 shots of ultrasonic compression waves at a rate of between 100 and 100 000 shots per second,
- detecting and recording in real time the echoes generated by the reflecting particles of the viscoelastic medium at each ultrasonic wave shot, these echoes corresponding to successive images of the observed medium,

and **in that** said method furthermore comprises a subsequent image processing step in the course of which the images thus obtained are processed at a later time at least by cross-correlation between successive images, so as to determine at each point of the observation field a motion parameter chosen between the displacement and the strain of the viscoelastic medium, in such a way as to thus obtain a succession of images showing the evolution of the motion parameter of the viscoelastic medium under the effect of the propagation of the shear wave.

**2.** The method as claimed in claim 1, in which the duration of the low-frequency pulse is between 1/2f and 2/f.

**3.** The method as claimed in claim 1 or claim 2, in which the central frequency of the low-frequency pulse is between 30 and 1000 Hz.

4. The method as claimed in any one of the preceding claims, in which the observed viscoelastic medium (1) consists of a living body comprising at least one internal organ subjected to pulsatile motions, the low-frequency pulse which generates the shear wave being constituted by a pulsatile motion of said internal organ.

5. The method as claimed in any one of claims 1 to 3, in which the observed viscoelastic medium (1) is delimited by an outside surface (3) and the low-frequency pulse is applied in the vicinity of this outside surface.

6. The method as claimed in claim 5, in which the low-frequency pulse is applied by a means of excitation chosen from:

- an acoustic wave generated by at least one acoustic transducer (2),
- and a shock generated locally by physical contact in the vicinity of the outside surface (3) of the viscoelastic medium.

7. The method as claimed in claim 5 or claim 6, in which the ultrasonic compression wave shots are emitted and the echoes generated by the reflecting particles of the viscoelastic medium are detected by means of a bank of transducers (6) which comprises at least one transducer (T1, ..., Tn) and which is arranged in contact with the outside surface of the viscoelastic medium, the shear wave being applied to the viscoelastic medium by imposing a pulsatile displacement on said bank of transducers.

8. The method as claimed in claim 7, in which said motion parameter is the strain of the viscoelastic medium.

9. The method as claimed in any one of the preceding claims in which, in the course of the observation of the propagation of the shear wave, between 100 and 10 000 ultrasonic compression wave shots are emitted at a rate of between 1000 and 100 000 shots per second.

10. The method as claimed in any one of the preceding claims, in which the observation field extends at least along a plane comprising on the one hand, the first axis (X) and on the other hand, a second axis perpendicular to the first axis (Y).

11. The method as claimed in claim 10, in which, in the course of the propagation observation step, a bank (6) of several acoustic transducers (T1, ..., Tn) arranged at least along the second axis (Y) is used to emit the ultrasonic acoustic compression wave shots and detect the echoes generated by the reflecting particles (5) of the viscoelastic medium, the echoes detected by each acoustic transducer being stored directly without prior processing in the course of the propagation observation step, and the image processing step comprising a preliminary substep of forming paths in the course of which an image of the viscoelastic medium corresponding to each ultrasonic compression wave shot is generated by combining at least some of the echoes received by the various transducers (T1, ..., Tn).

12. The method as claimed in any one of the preceding claims, in which the image processing step is followed by a viewing step in the course of which a film consisting of the succession of processed images is viewed under slow motion, each point of each image exhibiting an optical parameter which varies according to the value of the motion parameter assigned to this point.

13. The method as claimed in claim 12, in which the optical parameter is chosen from the gray level and the chromatic level.

14. The method as claimed in any one of the preceding claims, in which the image processing step is followed by a mapping step in the course of which, on the basis of the evolution of the motion parameter over the course of time in the observation field, at least one propagation parameter is calculated for the shear wave at at least some points of the observation field.

15. The method as claimed in claim 14, in which the shear wave propagation parameter which is calculated in the course of the mapping step is chosen from the speed of the shear waves, the shear modulus, the attenuation of the shear waves, the shear elasticity, and the shear viscosity.

16. An imaging device using shear waves to observe a diffusing viscoelastic medium (1) which contains particles (5) reflecting ultrasonic compression waves, this device comprising means of excitation (2) for generating an elastic shear wave in the viscoelastic medium and means of acquisition (CPU, Ti, Ei, Mi) for observing, by means of at least one ultrasonic compression wave, the displacement of the viscoelastic medium subjected to said shear wave,

**characterized in that** the means of excitation (2) are adapted to apply to the viscoelastic medium (1) an excitation having the form of a low-frequency pulse which exhibits a central frequency f of between 20 and 5000 Hz, this low-frequency pulse exhibiting a duration of between 1/2f and 20/f,

and **in that** the means of acquisition (CPU, Ti, Ei, Mi) are adapted to observe the propagation of the shear wave simultaneously at a multitude of points in the observed medium, these points forming a substantially continuous observation field extending at least along a first axis (X), said means of acquisition being adapted for:

- emitting into the observed medium a succession of at least 10 shots of ultrasonic compression waves at a rate of between 100 and 100 000 shots per second,
- detecting and recording in real time the echoes generated by the reflecting particles of the viscoelastic medium at each ultrasonic wave shot, these echoes corresponding to successive images of the observed medium,

and **in that** said device furthermore comprises image processing means (CPU, S DSP) adapted to process at a later time the images obtained by the observation means, at least by cross-correlation between successive images, so as to determine at each point of the observation field a motion parameter chosen between the displacement and the strain of the viscoelastic medium, in such a way as to thus obtain a succession of images showing the evolution of the motion parameter of the viscoelastic medium under the effect of the propagation of the shear wave.

**17.** The device as claimed in claim 16, in which the means of observation comprise a bank (6) of transducers (T1, ..., Tn) which includes at least one transducer and which is adapted to be arranged in contact with an outside surface (3) delimiting the viscoelastic medium (1), the means of excitation (2) being adapted to impose a pulsatile displacement on said bank (6) of transducers.

**18.** The device as claimed in claim 17, in which said motion parameter is the strain of the viscoelastic medium.

**19.** The device as claimed in any one of claims 17 and 18, in which the observation field extends at least along a plane comprising on the one hand, the first axis (X) and on the other hand, a second axis (Y) perpendicular to the first axis, the bank (6) of transducers comprising several transducers (T1, ..., Tn) arranged at least along the second axis, control means (4, CPU) being provided for selectively operating the device either in the mode of imaging by shear waves, or in a standard echography mode making it possible to acquire between 10 and 100 images per second.

**Patentansprüche**

**1.** Verfahren zur Bilderzeugung durch Verwendung von Scherwellen, um ein streuendes viskoelastisches Medium (1) zu beobachten, das die Ultraschalldruckwellen reflektierende Teilchen (5) enthält, ein Verfahren, bei dem eine elastische Scherwelle im viskoelastisches Medium erzeugt und mittels mindestens einer Ultraschalldruckwelle die Verschiebung des der Scherwelle ausgesetzten viskoelastischen Mediums (1) beobachtet wird,
**dadurch gekennzeichnet, dass** die Scherwelle erzeugt wird, indem an das viskoelastische Medium eine Anregung in Form eines niederfrequenten Impulses angelegt wird, der eine zentrale Frequenz f zwischen 20 und 5000 Hz aufweist, wobei dieser niederfrequente Impuls eine Dauer zwischen 1/2f und 20/f hat,
dass es einen Schritt der Beobachtung der Ausbreitung umfasst, während dem die Ausbreitung der Scherwelle gleichzeitig an vielen Punkten im beobachteten Medium beobachtet wird, wobei diese Punkte ein im Wesentlichen kontinuierliches Beobachtungsfeld bilden, das sich mindestens entlang einer ersten Achse (X) erstreckt, wobei dieser Schritt der Beobachtung der Ausbreitung der Scherwelle darin besteht:

- in das beobachtete Medium eine Folge von wenigstens 10 Ultraschalldruckwellenschüssen mit einer Taktfolge von zwischen 100 und 100 000 Schüssen pro Sekunde abzugeben,
- die von den reflektierenden Teilchen des viskoelastischen Mediums bei jedem Ultraschallwellenschuss erzeugten Echos in Echtzeit zu erfassen und aufzuzeichnen, wobei diese Echos aufeinander folgenden Bildern des beobachteten Mediums entsprechen,

und dass das Verfahren außerdem einen weiteren Schritt der Bildverarbeitung aufweist, während dem die so erhaltenen Bilder zeitverzögert mindestens durch Interkorrelation zwischen aufeinander folgenden Bildern verarbeitet werden, um an jedem Punkt des Beobachtungsfelds einen Bewegungsparameter zu bestimmen, der aus der Verschiebung und der Verformung des viskoelastischen Mediums ausgewählt wird, um so eine Folge von Bildern zu erhalten, welche die Entwicklung des Bewegungsparameters des viskoelastischen Mediums unter der Einwirkung der Ausbreitung der Scherwelle aufzeigt.

**2.** Verfahren nach Anspruch 1, bei dem die Dauer des niederfrequenten Impulses zwischen 1/2f und 2/f liegt.

**3.** Verfahren nach Anspruch 1 oder Anspruch 2, bei dem die zentrale Frequenz des niederfrequenten Impulses zwischen 30 und 1000 Hz liegt.

**4.** Verfahren nach einem der vorhergehenden Ansprüche, bei dem das beobachtete viskoelastische Medium (1) aus einem lebenden Körper besteht, der mindestens ein inneres Organ aufweist, das Impulsbewegungen unterworfen ist, wobei der die Scherwelle erzeugende niederfrequente Impuls aus einer Impulsbewegung des inneren Organs besteht.

**5.** Verfahren nach einem der Ansprüche 1 bis 3, bei dem das beobachtete viskoelastische Medium (1) von einer Außenfläche (3) begrenzt wird, und der niederfrequente Impuls in Höhe dieser Außenfläche angelegt wird.

**6.** Verfahren nach Anspruch 5, bei dem der niederfrequente Impuls durch ein Anregungsmittel angelegt wird, das ausgewählt wird aus:

- einer von mindestens einem akustischen Wandler (2) erzeugten Schallwelle,
- und einem Stoß, der lokal durch physikalischen Kontakt in Höhe der Außenfläche (3) des viskoelastischen Mediums erzeugt wird.

**7.** Verfahren nach Anspruch 5 oder Anspruch 6, bei dem die Ultraschalldruckwellenschüsse abgegeben und die von den reflektierenden Teilchen des viskoelastischen Mediums erzeugten Echos von einem Satz von Wandlern (6) erfasst werden, der mindestens einen Wandler (T1, ..., Tn) enthält, und der in Kontakt mit der Außenfläche des viskoelastischen Mediums angeordnet ist, wobei die an das viskoelastische Medium angelegte Scherwelle in dem Satz von Wandlern eine Impulsverschiebung bewirkt.

**8.** Verfahren nach Anspruch 7, bei dem der Bewegungsparameter die Verformung des viskoelastischen Mediums ist.

**9.** Verfahren nach einem der vorhergehenden Ansprüche, bei dem während der Beobachtung der Ausbreitung der Scherwelle zwischen 100 und 10 000 Ultraschalldruckwellenschüsse mit einer Taktfolge von zwischen 1000 und 100 000 Schüssen pro Sekunde abgegeben werden.

**10.** Verfahren nach einem der vorhergehenden Ansprüche, bei dem das Bebachtungsfeld sich mindestens gemäß einer Ebene erstreckt, die einerseits die erste Achse (X) und andererseits eine zweite Achse (Y) lotrecht zur ersten Achse enthält.

**11.** Verfahren nach Anspruch 10, bei dem während des Schritts der Beobachtung der Ausbreitung ein Satz (6) von mehreren akustischen Wandlern (T1, ..., Tn) verwendet wird, die mindestens gemäß der zweiten Achse (Y) angeordnet sind, um die Ultraschalldruckwellenschüsse abzugeben und die Echos zu erfassen, die von den reflektierenden Teilchen (5) des viskoelastischen Mediums erzeugt werden, wobei die von jedem akustischen Wandler erfassten Echos direkt ohne vorherige Verarbeitung während des Schritts der Beobachtung der Ausbreitung gespeichert werden, und der Bildverarbeitungsschritt einen vorhergehenden Unterschritt der Bildung von Kanälen aufweist, während dem durch Kombination mindestens bestimmter der Echos, die von den verschiedenen Wandlern (T1, ..., Tn) empfangen werden, ein Bild des viskoelastischen Mediums erzeugt wird, das jedem Schuss einer Ultraschalldruckwelle entspricht.

**12.** Verfahren nach einem der vorhergehenden Ansprüche, bei dem auf den Bildverarbeitungsschritt ein Anzeigeschritt folgt, während dem in Zeitlupe ein Film gezeigt wird, der aus einer Folge der verarbeiteten Bilder besteht, wobei jeder Punkt jedes Bildes einen optischen Parameter repräsentiert, der sich entsprechend dem Wert des diesem Punkt zugeordneten Bewegungsparameters ändert.

**13.** Verfahren nach Anspruch 12, bei dem der optische Parameter aus einem Graupegel und einem chromatischen Pegel ausgewählt wird.

**14.** Verfahren nach einem der vorhergehenden Ansprüche, bei dem auf den Bildverarbeitungsschritt ein Kartographieschritt folgt, während dem ausgehend von der zeitlichen Entwicklung des Bewegungsparameters im Beobachtungsfeld mindestens ein Ausbreitungsparameter der Scherwelle an mindestens bestimmten Punkten des Beobachtungsfelds berechnet wird.

**15.** Verfahren nach Anspruch 14, bei dem der Ausbreitungsparameter der Scherwelle, der während des Kartographieschritts berechnet wird, aus der Geschwindigkeit der Scherwellen, dem Schermodul, der Dämpfung der Scherwellen, der Scherelastizität und der Scherviskosität ausgewählt wird.

**16.** Vorrichtung zur Bilderzeugung durch Verwendung von Scherwellen, um ein streuendes viskoelastisches Medium (1) zu beobachten, das die Ultraschalldruckwellen reflektierende Teilchen (5) enthält, wobei diese Vorrichtung Anregungsmittel (2) zur Erzeugung einer elastische Scherwelle im viskoelastischen Medium und Erfassungsmittel (CPU; Ti, Ei, Mi) aufweist, um mittels mindestens einer Ultraschalldruckwelle die Verschiebung des der Scherwelle ausgesetzten viskoelastischen Mediums zu beobachten,

**dadurch gekennzeichnet, dass** die Anregungsmittel (2) ausgelegt sind, um an das viskoelastische Medium (1) eine Anregung in Form eines niederfrequenten Impulses anzulegen, der eine zentrale Frequenz f aufweist, die zwischen 20 und 5000 Hz liegt, wobei dieser niederfrequente Impuls eine Dauer zwischen 1/2f und 20/f hat,

dass die Erfassungsmittel (CPU, Ti, Ei, Mi) ausgelegt sind, um die Ausbreitung der Scherwelle gleichzeitig an einer Vielzahl von Punkten im beobachteten Medium zu beobachten, wobei diese Punkte ein im Wesentlichen kontinuierliches Beobachtungsfeld bilden, das sich mindestens gemäß einer ersten Achse (X) erstreckt, wobei die Erfassungsmittel ausgelegt sind, um:

- im beobachteten Medium eine Folge von mindestens 10 Ultraschalldruckwellenschüssen mit einer Taktfolge zwischen 100 und 100 000 Schüssen pro Sekunde abzugeben,
- die von den reflektierenden Teilchen des viskoelastischen Mediums bei jedem Ultraschallwellenschuss erzeugten Echos in Echtzeit zu erfassen und aufzuzeichnen, wobei diese Echos aufeinander folgenden Bildern des beobachteten Mediums entsprechen,

und dass die Vorrichtung außerdem Bildverarbeitungsmittel (CPU, S, DSP) aufweist, die ausgelegt sind, um zeitverzögert die ausgehend von den Beobachtungsmitteln erhaltenen Bilder mindestens durch Interkorrelation zwischen aufeinander folgenden Bildern zu verarbeiten, um an jedem Punkt des Beobachtungsfelds einen Bewegungsparameter zu bestimmen, der aus der Verschiebung und der Verformung des viskoelastischen Mediums ausgewählt wird, um so eine Folge von Bildern zu erhalten, die die Entwicklung des Bewegungsparameters des viskoelastischen Mediums unter der Wirkung der Ausbreitung der Scherwelle zeigen.

**17.** Vorrichtung nach Anspruch 16, bei der die Beobachtungsmittel einen Satz (6) von Wandlern (Tl, ..., Tn) aufweisen, der mindestens einen Wandler enthält und ausgelegt ist, um in Kontakt mit einer das viskoelastische Medium (1) begrenzenden Außenfläche (3) angeordnet zu werden, wobei die Anregungsmittel (2) ausgelegt sind, um dem Satz (6) von Wandlern eine Impulsverschiebung aufzuzwingen.

**18.** Vorrichtung nach Anspruch 17, bei der der Bewegungsparameter die Verformung des viskoelastischen Mediums ist.

**19.** Vorrichtung nach einem der Ansprüche 17 und 18, bei dem das Beobachtungsfeld sich mindestens gemäß einer Ebene erstreckt, die einerseits die erste Achse (X) und andererseits eine zweite Achse (Y) lotrecht zur ersten Achse aufweist, wobei der Satz (6) von Wandlern mehrere Wandler (T1, ..., Tn) enthält, die gemäß mindestens der zweiten Achse angeordnet sind, wobei Steuermittel (4, CPU) vorgesehen sind, um die Vorrichtung selektiv entweder im Modus der Bilderzeugung durch Scherwellen oder in einem Standard-Echographiemodus arbeiten zu lassen, der es ermöglicht, zwischen 10 und 100 Bilder pro Sekunde zu erfassen.

# FIG.1.

# FIG.2.